(19) Europäisches Patentamt
European Patent Office
Office européen des brevets

(11) Veröffentlichungsnummer: **0 346 745 A1**

(12) **EUROPÄISCHE PATENTANMELDUNG**

(21) Anmeldenummer: **89110264.2**

(22) Anmeldetag: **07.06.89**

(51) Int. Cl.4: **C07D 471/04 , C07D 471/14 , C07D 243/38 , C07D 487/04 , C07D 495/04 , C07D 495/14 , C07D 403/12 , A61K 31/55**

Patentansprüche für folgende Vertragsstaaten: ES + GR.

(30) Priorität: **15.06.88 DE 3820346**

(43) Veröffentlichungstag der Anmeldung: **20.12.89 Patentblatt 89/51**

(84) Benannte Vertragsstaaten: **AT BE CH DE ES FR GB GR IT LI LU NL SE**

(71) Anmelder: **Dr. Karl Thomae GmbH**
**Postfach 1755**
**D-7950 Biberach 1(DE)**

(72) Erfinder: **Mihm, Gerhard, Dr. Dipl.-Chem.**
**Nickeleshalde 5/1**
**D-7950 Biberach 1(DE)**
Erfinder: **Eberlein, Wolfgang, Dr. Dipl.-Chem.**
**Obere Au 6**
**D-7950 Biberach 1(DE)**
Erfinder: **Engel, Wolfhard, Dr. Dipl.-Chem.**
**Mozartstrasse 13**
**D-7950 Biberach 1(DE)**
Erfinder: **Trummlitz, Günter, Dr. Dipl.-Chem.**
**Buchenweg 27**
**D-7951 Warthausen(DE)**
Erfinder: **Mayer, Norbert Dr.**
**Friedrich-Ebert-Strasse 66**
**D-7950 Biberach 1(DE)**
Erfinder: **de Jonge, Adriaan, Dr.**
**De Boomgaard 19**
**NL-3971 LD Driebergen(NL)**
Erfinder: **Doods, Henri, Dr.**
**Hornsteinweg 7**
**D-7951 Warthausen(DE)**

(54) **Neue kondensierte Diazepinone, Verfahren zu ihrer Herstellung und diese Verbindungen enthaltende Arzneimittel.**

(57) Beschrieben werden neue kondensierte Diazepinone der allgemeinen Formel I,

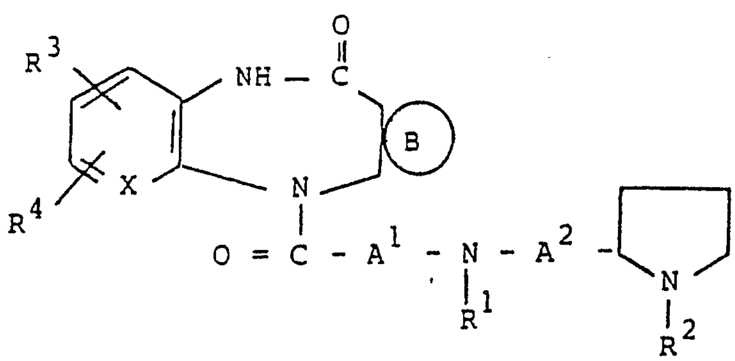

, (I)

in der 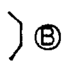 einen der zweiwertigen Reste

EP 0 346 745 A1

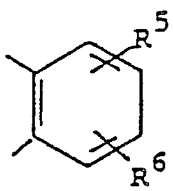 , 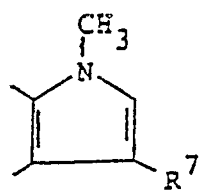 , 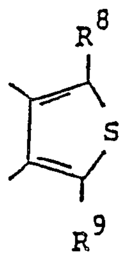 , 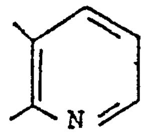 oder 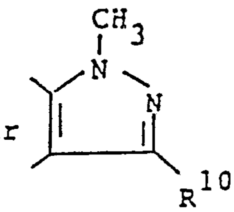

(S) (T) (U) (V) (W)

darstellt
X eine =CH-Gruppe oder ein Stickstoffatom,
$A^1$ und $A^2$ niedere Alkylenreste,
$R^1$ und $R^2$ Wasserstoff, niedere Alkyl- oder Cycloalkylreste,
$R^3$ niedere Alkylreste, Chlor oder Wasserstoff,
$R^4$ Wasserstoff oder Methyl, $R^5$ und $R^6$ Halogen, niedere Alkylreste oder gegebenenfalls auch Wasserstoff, $R^2$ Wasserstoff, Chlor oder Methyl, $R^8$ und $R^9$ Wasserstoff oder niedere Alkylreste, $R^9$ zusätzlich ein Halogenatom, und $R^{10}$ Wasserstoff oder Methyl bedeuten und ihre Säureadditionssalze und ihre möglichen Enantiomeren; des weiteren werden Verfahren zur Herstellung dieser Verbindungen beschrieben.

Die Verbindungen eignen sich als vagale Schrittmacher zur Behandlung von Bradycardien und Bradyarrhythmien, sie üben spasmolytische Wirkungen auf periphere Organe, insbesondere Colon, Blase und Bronchien, aus.

## Neue kondensierte Diazepinone, Verfahren zu ihrer Herstellung und diese Verbindungen enthaltende Arzneimittel

Die Erfindung betrifft neue kondensierte Diazepinone, Verfahren zu ihrer Herstellung und diese Verbindungen enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 ist beschrieben, daß durch Einführung neuartiger Aminoacylreste gegenüber den Verbindungen der obengenannten Publikationen völlig andersartige, wertvolle pharmakologische Eigenschaften induziert werden können. Gegenüber diesen Verbindungen zeichnen sich die erfindungsgemäßen kondensierten Diazepinone bei vergleichbarer oder verbesserter Selektivität überraschenderweise durch eine wesentlich verstärkte Wirkung und Resorption nach oraler Gabe aus.

Die neuen kondensierten Diazepinone besitzen die allgemeine Formel I,

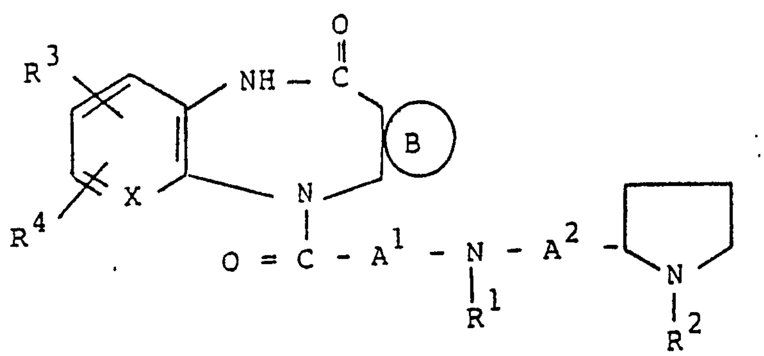

, (I)

in der 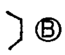 einen der zweiwertigen Reste

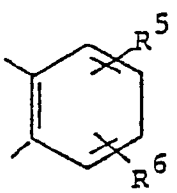 , 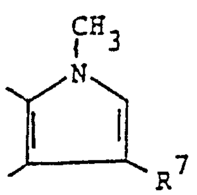 , 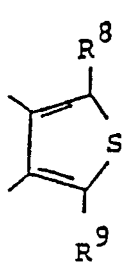 , 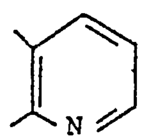 oder 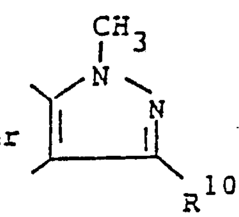

(S) (T) (U) (V) (W)

darstellt und

X, $A^1$, $A^2$ und $R^1$ bis $R^{10}$ die folgenden Bedeutungen besitzen:

X stellt eine =CH-Gruppe oder ein Stickstoffatom dar;

$A^1$ und $A^2$ sind geradkettige, gesättigte Alkylenreste mit 1 bis 4 Kohlenstoffatomen;

$R^1$ und $R^2$ sind Wasserstoffatome oder verzweigte oder unverzweigte Alkylreste mit 1 bis 4 Kohlenstoffatomen oder Cycloalkylreste mit 4 bis 7 Kohlenstoffatomen, die gegebenenfalls noch durch eine Hydroxygruppe substituiert sein können;

$R^3$ ist eine Alkylgruppe mit 1 bis 4 C-Atomen, ein Chloratom oder ein Wasserstoffatom;

$R^4$ ein Wasserstoffatom oder eine Methylgruppe;

$R^5$ und $R^6$ bedeuten jeweils ein Fluor-, Chlor- oder Bromatom, eine Alkylgruppe mit 1 bis 4 C-Atomen oder, für den Fall, daß X eine =CH-Gruppe darstellt, auch ein Wasserstoffatom,

sofern X ein Stickstoffatom, $A^2$ eine geradkettige, gesättigte Alkylengruppe mit 2 bis 4 C-Atomen und $R^1$ ein Wasserstoffatom bedeuten, muß mindestens einer der Reste $R^5$ oder $R^6$ eine der obengenannten Bedeutungen mit Ausnahme der eines Wasser- stoffatoms innehaben, besitzt aber $R^1$ eine der obengenannten Bedeutungen mit Ausnahme der eines Wasserstoffatoms und ist $A^2$ eine geradkettige, gesättigte Alkylengruppe mit 2 bis 4 C-Atomen, so können die Reste $R^5$ und $R^6$ alle oben genannten Bedeutungen

innehaben;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und
$R^{10}$ ein Wasserstoffatom oder eine Methylgruppe.

Für den Fall, daß ] Ⓑ den zweiwertigen Rest (T) darstellt und $R^7$ ein Wasserstoffatom ist, kann $R^3$ kein Chloratom bedeuten; für den Fall, daß ] Ⓑ den zweiwertigen Rest (V) darstellt, kann X kein Stickstoffatom bedeuten.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bern steinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende bevorzugte Verbindungen genannt:
5,11-Dihydro-11-[[[2-(1-methyl-2-pyrrolidinyl)ethyl]methylamino]acetyl]-6H-pyrido[2,3-b][1,4]benzodiazepin-6-on,
9-Chlor-5,11-dihydro-11-[[[2-(1-methyl-2-pyrrolidinyl)ethyl]ethylamino]acetyl]-6H-pyrido[2,3-b][1,4]-benzodiazepin-6-on und
5,10-Dihydro-5-[[[2-(1-methyl-2-pyrrolidinyl)ethyl]-methylamino]acetyl]-11H-dibenzo[b,e][1,4]diazepin-11-on
und deren physiologisch verträgliche Salze mit anorganischen oder organischen Säuren.

Erfindungsgemäß erhält man die neuen basisch substituierten kondensierten Diazepinone der allgemeinen Formel I nach folgenden Verfahren:

a) Basisch substituierte kondensierte Diazepinone der allgemeinen Formel Ia,

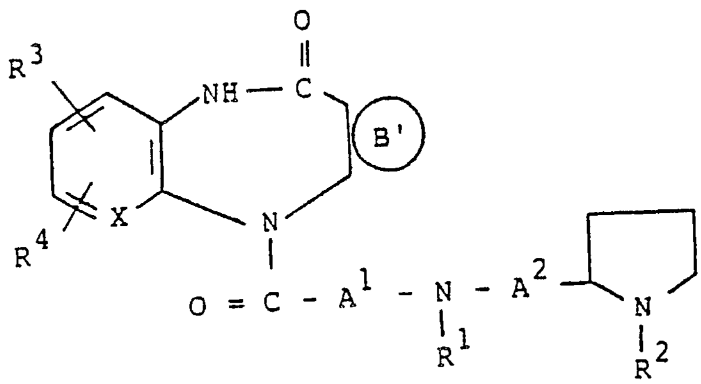

,(Ia)

in der
X, $A^1$, $A^2$ und $R^1$ bis $R^4$ wie oben angegeben definiert sind und ] Ⓑ' einen der zweiwertigen Reste (S), (U), (V), (W) oder (T')

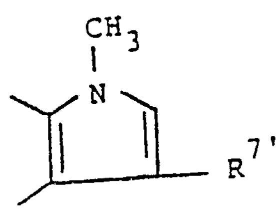

(T')

darstellt, worin $R^{7'}$ ein Chloratom oder eine Methylgruppe ist,
erhält man durch Umsetzung von Halogenacylverbindungen der allgemeinen Formel II,

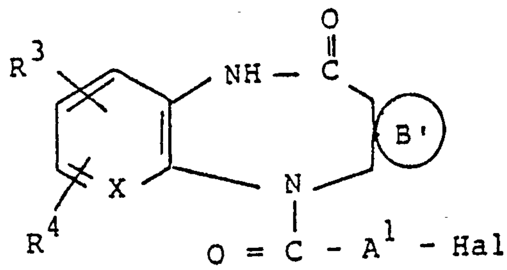

,(II)

in der X, A', $R^3$, $R^4$ und 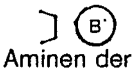 die vorstehend genannten Bedeutungen haben und Hal ein Chlor-, Brom- oder Iodatom ist, mit Aminen der allgemeinen Formel III,

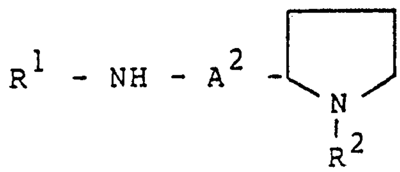

,(III)

worin $R^1$, $R^2$ und $A^2$ die genannten Bedeutungen haben.

Die Aminierung erfolgt in einem inerten Lösungsmittel bei Temperaturen zwischen -10°C und der Siedetemperatur des Lösungsmittels, vorzugsweise entweder mit wenigstens 2 Molen sekundärem Amin der allgemeinen Formel III oder mit 1 bis 2 Molen eines sekundären Amins der allgemeinen Formel III und einer Hilfsbase. Als Lösungsmittel kommen beispielsweise chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone, wie Aceton; Acetonitril, Dimethylformamid oder 1,3-Dimethyl-2-imidazolidinon in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall-oder Erdalkalimetallcarbonate oder -hydrogencarbonate, -hydroxide oder -oxide genannt. Gegebenenfalls kann die Reaktion durch Zusatz von Alkalimetalliodiden beschleunigt werden. Die Reaktionszeiten betragen je nach Menge und Art des eingesetzten Amins der allgemeinen Formel III zwischen 15 Minuten und 80 Stunden.

b.) Dieselben basisch substituierten kondensierten Diazepinone der allgemeinen Formel Ia erhält man auch durch Acylierung von Diazepinonen der allgemeinen Formel IV,

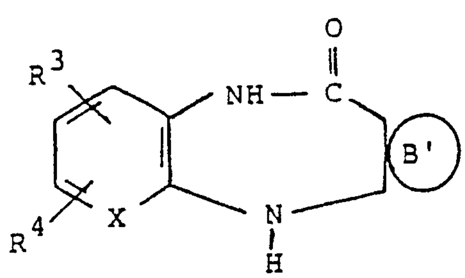

,(IV)

worin X, $R^3$, $R^4$ und 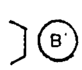 die oben angegebenen Bedeutungen haben, mit Carbonsäurederivaten der allgemeinen Formel V,

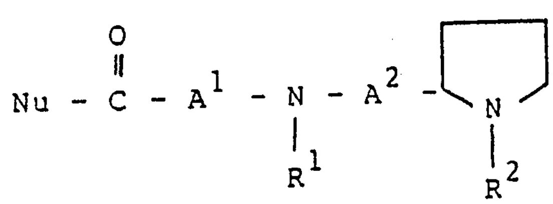

,(V)

worin $A^1$, $A^2$, $R^1$ und $R^2$ die oben angegebenen Bedeutungen haben und Nu eine nucleofuge Gruppe bzw. Abgangsgruppe darstellt.

Die Umsetzung der Verbindungen der allgemeinen Formel IV mit den Säurederivaten der allgemeinen Formel V erfolgt in an sich bekannter Weise. Die Abgangsgruppe Nu ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte Anhydride, wie sie aus Salzen der entsprechenden Säuren (Nu = OH) und Säurechloriden, wie Phosphoroxidchlorid, Diphosphorsäuretetrachlorid oder Chlorameisensäureestern gebildet werden oder die bei der Umsetzung von Verbindungen der allgemeinen Formel V (Nu = OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien bei spielsweise Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-(Dimethylamino)pyridin, oder Natriumhydrid genannt. Die Reaktion wird bei Temperaturen zwischen -25°C und 130°C in einem inerten Lösungsmittel durchgeführt. Als inerte Lösungsmittel kommen beispielsweise chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon in Frage. Die Reaktionszeiten betragen je nach Menge und Art des eingesetzten Acylierungsmittels der allgemeinen Formel V zwischen 15 Minuten und 80 Stunden. Es ist nicht nötig, die Verbindungen der allgemeinen Formel V in reiner Form herzustellen, sie können vielmehr im Reaktionsansatz in bekannter Weise in situ erzeugt werden.

c) Die unter die allgemeine Formel I fallenden neuen Pyrrolo-kondensierten Diazepinone der allgemeinen Formel Ib,

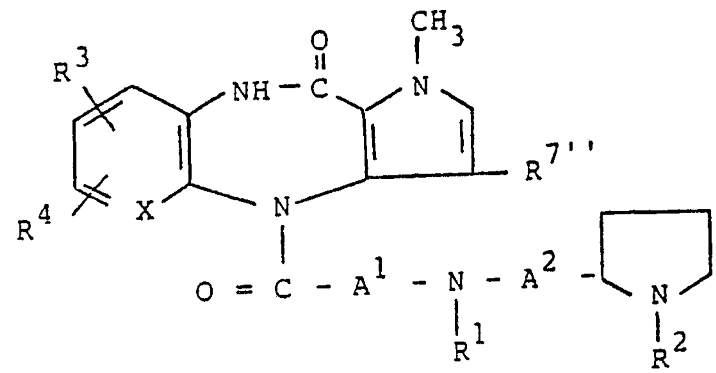

,(Ib)

worin

X, $A^1$, $A^2$ und $R^1$ bis $R^4$ die eingangs erwähnten Bedeutungen haben und $R^{7''}$ ein Wasserstoffatom darstellt, können durch Hydrogenolyse aus solchen Verbindungen der allgemeinen Formel Ib hergestellt werden, in denen $R^{7''}$ ein Chloratom bedeutet.

Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von 0°C bis 130°C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110°C, sowie die Reduktion mit Triethylammoniumformiat in Gegenwart überschüssigen

Triethylamins und von Palladium auf Tierkohle oder von Palladiumacetat und Triarylphosphinen, wie Triphenylphosphin, Tris-(o-tolyl)phosphin, Tris-(2,5-diisopropylphenyl)phosphin, bei Temperaturen zwischen 40 und 110 °C, bewährt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Die erfindungsgemäßen basisch substituierten kondensierten Diazepinone der allgemeinen Formel I enthalten, insbesondere wenn 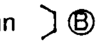 den zweiwertigen Rest (U) darstellt, bis zu zwei unabhängige chirale Elemente. Neben dem asymmetrischen Kohlenstoffatom in der Seitenkette ist als weiteres chirales Element der acylierte Tricyclus selbst anzusehen, der in zwei spiegelbildlichen Formen vorliegen kann. Von der Natur des Tricyclus hängt es ab, ob die Energiebarriere für eine Inversion an diesem Zentrum so hoch ist, daß die einzelnen Isomeren bei Zimmertemperatur stabil sind und isolierbar werden. Es hat sich gezeigt, daß bei Verbindungen der allgemeinen Formel I, worin X ein Stickstoffatom ist und die dem Diazepinon-Ring benachbarten Positionen unsubstituiert sind, die erforderliche Aktivierungsenergie so stark vermindert ist, daß Diastereomere bei Zimmertemperatur nicht mehr nachgewiesen geschweige denn präparativ isoliert werden können.

Die erfindungsgemäßen aminoacylierten kondensierten Diazepinone der allgemeinen Formel I enthalten also bis zu zwei Chiralitätselemente, von denen eines bei Zimmertemperatur unter Umständen nicht konfigurationsstabil ist. Solche Verbindungen können deshalb in mehreren diastereomeren und/oder jeweils als enantiomere (+)- und (-)-Formen auftreten. Die Erfindung umfaßt die einzelnen Isomeren ebenso wie ihre Gemische. Die Trennung der jeweiligen Diastereomeren gelingt auf Grund der unterschiedlichen physiko-chemischen Eigenschaften, z.B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit nur einem Enantiomeren der allgemeinen Formel III bzw. V durchführt.

Die Halogenacylverbindungen der allgemeinen Formel II werden nach bekannten Methoden hergestellt (vgl. U.S.-Patent Nr. 4.550.107).

Zwischenverbindungen der allgemeinen Formel III lassen sich nach dem Fachmann geläufigen Methoden leicht synthetisieren, z. B. durch Reduktion entsprechender Pyrrolidincarbonsäurealkylamide mit Lithiumaluminiumhydrid oder Diboran.

Die Ausgangsverbindungen der allgemeinen Formel V, in der Nu eine Alkoxygruppe bedeutet, erhält man durch Umsetzung von Diaminen der allgemeinen Formel III mit Halogencarbonsäureestern, gegebenenfalls unter Verwendung zusätzlicher Hilfsbasen, z.B. Triethylamin, oder Katalysatoren, beispielsweise Triton B. Durch Verseifung der erhaltenen Ester, z.B. mit Barytlauge, erhält man die unter die allgemeine Formel V fallenden Carbonsäuren, die zur Herstellung von Derivaten mit anderen nucleofugen Gruppen dienen können.

Ein weiterer Gegenstand der Erfindung sind Arzneimittel, die ein oder mehrere kondensierte Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht die gegebenenfalls in Form mehrerer, vorzugsweise 1 bis 3 Einzelgaben, zur Erzielung der gewünschten Ergebnisse

verabreicht wird.

Die basisch substituierten kondensierten Diazepinone der allgemeinen Formel I und ihre Säureadditionssalze besitzen wertvolle Eigenschaften; insbesondere besitzen sie günstige Effekte auf die Herzfrequenz und sind angesichts fehlender magensäuresekretionshemmender, salivationshemmender und mydriatischer Einflüsse als vagale Schrittmacher zur Behand lung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon, Blase und Bronchien.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Bindungsstudien an muscarinischen Rezeptoren:

Bestimmung des $IC_{50}$-Wertes in vitro

Als Organspender dienten männliche Sprague-Dawley-Ratten mit 180-220 g Körpergewicht. Nach Entnahme von Herz, Submandibularis und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 400 |
| Großhirnrinde | 1:3000 |
| Submandibularis | 1: 400 |

Die Inkubation der Organhomogenate erfolgte bei einer bestimmten Konzentration des Radioliganden und einer Konzen trationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Quinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle 1 zu ersehen.

B. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

1. $M_1$-/$M_2$-Selektivität an der Ratte
2. Speichselsekretionshemmende Wirkung an der Ratte

3. Hemmung der Acetylcholinwirkung auf Blase, Bronchien und Herzfrequenz des Meerschweinchens untersucht.

1. $M_1$-$M_2$-Selektivität an der Ratte

Die angewandte Methode wurde von Hammer und Giachetti (Life Sciences 31, 2991-2998 (1982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle II.

2. Speichselsekretionshemmende Wirkung an der Ratte

Nach Lavy und Mulder (Arch. int. Pharmacodyn. 178, 437-445, (1969)) erhielten mit 1,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichel volumen um 50% verminderte, wurde graphisch ermittelt. Ergebnisse siehe Tabelle II.

3. Hemmung der Acetylcholinwirkung auf Blase, Bronchien und Herzfrequenz des Meerschweinchens

Narkotisierten Meerschweinchen wurden 5 Minuten nach Gabe der Prüfsubstanz 10 µg/kg Acetylcholin sowohl intravenös als auch gleichzeitig intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus -log $ED_{50}$-Werte bestimmt. Ergebnisse siehe Tabelle III.

Nach den vorstehenden Angaben wurden beispielsweise die folgenden Verbindungen untersucht:

A = 5,11-Dihydro-11-[[[2-(1-methyl-2-pyrrolidinyl)ethyl]methylamino]acetyl]-6H-pyrido[2,3-b][1,4]-benzodiazepin-6-on-hydrochlorid

B = 9-Chlor-5,11-dihydro-11-[[[2-(1-methyl-2-pyrrolidinyl)ethyl]ethylamino]acetyl]-6H-pyrido[2,3-b]-[1,4]benzodiazepin-6-on

C = 5,10-Dihydro-5-[[[2-(1-methyl-2-pyrrolidinyl)ethyl]methylamino]acetyl]-11H-dibenzo[b,e][1,4]-diazepin-11-on-hydrochlorid und als Vergleichssubstanzen

D = 11-[[2-[(Diethylamino)methyl]-1-piperidinyl]acetyl]-5,11-dihydro-6H-pyrido[2,3-b][1,4]-benzodiazepin-6-on (siehe US-Patent Nr. 4 550 107)

E = 5,11-Dihydro-11-[(4-methyl-1-piperazinyl)acetyl]-6H-pyrido[2,3-b][1,4]benzodiazepin-6-on (Pirenzepin, siehe US-Patent Nr. 3 660 380) und

F = Atropin

Tabelle I:

| Rezeptor-Bindungs-Tests, in vitro: | | | |
|---|---|---|---|
| Ergebnisse : | | | |
| | Rezeptor-Bindungs-Tests $IC_{50}[nMl^{-1}]$ | | |
| Substanz | Cortex | Herz | Submandibularis |
| A | 50 | 9 | 80 |
| B | 70 | 10 | 100 |
| C | 8 | 3 | 20 |
| D | 1200 | 140 | 5000 |
| E | 100 | 1500 | 200 |
| F | 2 | 4 | 4 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Großhirnrinde und Submandibularis.

Tabelle II:

| $M_1$-/$M_2$-Selektivität und Speichelsekretionshemmung an der Ratte: | | | |
|---|---|---|---|
| Ergebnisse: | | | |
| | -log $ED_{50}[Mkg^{-1}]$ | | |
| Substanz | Herz | Blutdruck | Salivation |
| A | 7,91 | 7,06 | 6,8 |
| D | 6,42 | 5,63 | 5,00 |
| E | 5,60 | 6,94 | 6,22 |
| F | 7,94 | 7,34 | 7,60 |

Tabelle III:

| Hemmung der Acetylcholinwirkung auf Blase, Bronchien und Herzfrequenz des Meerschweinchens: | | | |
|---|---|---|---|
| Ergebnisse: | | | |
| | -log $ED_{50}$[$Molkg^{-1}$] | | |
| Substanz | Herz | Bronchien | Blase |
| A | 7,7 | 7,6 | 6,85 |
| B | 7,0 | 6,72 | 6,08 |
| C | 7,54 | 7,53 | 6,63 |
| D | 5,84 | 5,58 | 4,73 |
| E | 5,85 | 6,57 | 5,36 |
| F | 7,70 | 7,96 | 7,03 |

Aus den pharmakologischen Daten der vorstehenden Tabellen II und III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichelsekretion beobachtet wird.

Außerdem deuten die pharmakologischen Daten der vorstehenden Tabelle III auf ein überraschend großes Unterscheidungsvermögen zwischen Herz und glatter Muskulatur.

Die genannten Substanzen weisen gegenüber der bereits bekannten Verbindung D eine wesentlich gesteigerte Wirkungsstärke auf. Dabei bleibt die therapeutisch nutzbare Selektivität erhalten. Dies führt zu einer geringeren Substanzbelastung des Patienten, ohne das Risiko muskarinischer Nebenwirkungen zu erhöhen.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Die nachfolgenden Beispiele sollen die Erfindung näher erläutern:
"Fp." bedeutet "Schmelzpunkt", "Z." bedeutet "Zersetzung". Für alle Verbindungen liegen befriedigende Elementaranalysen, IR-, UV-, $^1$H-NMR-, häufig auch Massenspektren vor.

## Beispiel 1

5,11-Dihydro-8-methyl-11-[[[2-(1-methyl-2-pyrrolidinyl)ethyl]amino]acetyl]-6H-pyrido[2,3-b][1,4]-benzodiazepin-6-on

9,0 g (0,03 mol) 11-(Chloracetyl)-5,11-dihydro-8-methyl-6H-pyrido[2,3-b][1,4]benzodiazepin-6-on, 3,2 g Natriumcarbonat und 5,1 g (0,04 mol) 2-(2-Aminoethyl)-1-methylpyrrolidin wurden in 120 ml absolutem Dioxan 2 Stunden lang unter Rückfluß gekocht. Es wurde heiß filtriert, das Lösungsmittel abgedampft und der Rückstand an Kieselgel säulenchromatographisch gereinigt (Fließmittel: Methylenchlorid/Methanol/Cyclohexan/Ammoniak 68/15/15/2 v/v/v/v). Das Eluat wurde aus Essigsäureethylester umkristallisiert.
Ausbeute: 1,9 g (16 % der Theorie)
Fp.: 107-109 °C (Essigsäureethylester).

## Beispiel 2

9-Chlor-5,11-dihydro-11-[[[2-(1-methyl-2-pyrrolidinyl)ethyl]amino]acetyl]-6H-pyrido[2,3-b][1,4]benzodiazepin-6-on

Hergestellt analog Beispiel 1 aus 9-Chlor-11-(chloracetyl)-5,11-dihydro-6H-pyrido[2,3-b][1,4]-benzodiazepin-6-on und 2-(2-Aminoethyl)-1-methylpyrrolidin in einer Ausbeute von 17 % der Theorie.
Fp.: 130-132 °C (Essigsäureethylester).

Beispiel 3

6,11-Dihydro-11-[[[2-(1-methyl-2-pyrrolidinyl)ethyl]amino]acetyl]-5H-pyrido[2,3-b][1,5]benzodiazepin-5-on-dihydrochlorid

Hergestellt analog Beispiel 1 aus 11-(Chloracetyl)-6,11-dihydro-5H-pyrido[2,3-b][1,5]benzodiazepin-5-on und 2-(2-Aminoethyl)-1-methylpyrrolidin. Die Base wurde durch Zugabe von wässriger Salzsäure in das Dihydrochlorid übergeführt.
Ausbeute: 14 % der Theorie.
Fp.: 206-207 °C (Isopropanol).

Beispiel 4

5,10-Dihydro-5-[[[2-(1-methyl-2-pyrrolidinyl)ethyl]amino]acetyl]-11H-dibenzo[b,e][1,4]diazepin-11-on-dihydrochlorid

Hergestellt analog Beispiel 1 aus 5-(Chloracetyl)-5,10-dihydro-11H-dibenzo[b,e][1,4]diazepin-11-on und 2-(2-Aminoethyl)-1-methylpyrrolidin. Die Base wurde mit konzentrierter Salzsäure in das Dihydrochlorid übergeführt.
Ausbeute: 13 % der Theorie.
Fp.: 215-217 °C (Isopropanol).

Beispiel 5

4,9-Dihydro-4-[[[2-(1-methyl-2-pyrrolidinyl)ethyl]amino]acetyl]-10H-thieno[3,4-b][1,5]benzodiazepin-10-on-dihydrochlorid

Hergestellt analog Beispiel 1 aus 4-(Chloracetyl)-4,9-dihydro-10H-thieno[3,4-b][1,5]benzodiazepin-10-on und 2-(2-Aminoethyl)-1-methylpyrrolidin. Die Base wurde mit konzentrierter Salzsäure in das Dihydrochlorid übergeführt.
Ausbeute: 20 % der Theorie.
Fp.: 223-225 °C (Isopropanol).

Beispiel 6

1,3-Dimethyl-4-[[[2-(1-methyl-2-pyrrolidinyl)ethyl]amino]acetyl]-1,4,9,10-tetrahydropyrazolo[4,3-e]pyrido[3,2-b]diazepin-10-on-difumarat

Hergestellt analog Beispiel 1 aus 4-(Chloracetyl)-1,3-dimethyl-1,4,9,10-tetrahydropyrazolo[4,3-e]pyrido-[3,2-b]diazepin-10-on und 2-(2-(Aminoethyl)-1-methylpyrrolidin. Die Base wurde mit Fumarsäure in das Fumarat übergeführt.
Ausbeute: 30 % der Theorie.
Fp.: 200-202 °C (Isopropanol).

Beispiel 7

5,11-Dihydro-11-[[[2-(1-ethyl-2-pyrrolidinyl)ethyl]amino]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 2-(2-Aminoethyl)-1-ethylpyrrolidin.
Ausbeute: 9,5 % der Theorie,
Fp.: 109-111 °C (Essigsäureethylester/Cyclohexan 2/1 v/v).

Beispiel 8

5,11-Dihydro-11-[3-[[2-(1-methyl-2-pyrrolidinyl)ethyl]-amino]propionyl]-6H-pyrido[2,3-b][1,4]benzodiazepin-6-on

Zu einer Lösung von 7,1 g (0,024 mol) 11-(3-Chlorpropionyl)-5,11-dihydro-6H-pyrido[2,3-b][1,4]-benzodiazepin-6-on und 3 g Triethylamin in 70 ml Dimethylformamid wurden bei Raumtemperatur 3,85 g (0,03 mol) 2-(2-Aminoethyl)-1-methylpyrrolidin getropft und noch 2 Stunden bei dieser Temperatur nachgerührt. Nach dem Abdampfen des Lösungsmittels wurde an Kieselgel säulenchromatographisch gereinigt (Mobile Phase: Ethylenchlorid/Methanol/Ammoniak 100/20/2 v/v/v). Der erhaltene amorphe Feststoff wurde nicht kristallin.
Ausbeute: 2,93 g (31 % der Theorie)
$R_F$ = 0,23 (Merck Fertigplatten, Kieselgel F 254; Fließmittel: Essigsäureethylester/Methanol/konz. Ammoniak 70/30/5 v/v/v).

Beispiel 9

5,10-Dihydro-5-[3-[[2-(1-methyl-2-pyrrolidinyl)ethyl]amino]propionyl]-11H-dibenzo[b,e][1,4]diazepin-11-on

Hergestellt analog Beispiel 8 aus 5-(3-Chlorpropionyl)-5,10-dihydro-11H-dibenzo[b,e][1,4]diazepin-11-on und 2-(2-Aminoethyl)-1-methylpyrrolidin als amorpher Feststoff.
Ausbeute: 13 % der Theorie.
$R_F$ = 0,34 (Merck Fertigplatten, Kieselgel F 254; Fließmittel: Essigsäureethylester/Methanol/konz. Ammoniak 70/30/5 v/v/v).

Beispiel 10

5,11-Dihydro-11-[[[2-(1-methyl-2-pyrrolidinyl)ethyl]methylamino]acetyl]-6H-pyrido[2,3-b][1,4]benzodiazepin-6-on-hydrochlorid

Eine Lösung von 28,7 g (0,1 mol) 11-(Chloracetyl)-5,11-dihydro-6H-pyrido[2,3-b][1,4]benzodiazepin-6-on und 15,7 g (0,11 mol) 1-Methyl-2-[2-(methylamino)ethyl]pyrrolidin in 300 ml absolutem Dimethylformamid wurde 8 Stunden bei Raumtemperatur gerührt. Nach dem Abdestillieren des Lösungsmittels wurde der Rückstand mit etwas Methanol verrieben. Die ausgefallenen Kristalle wurden abgesaugt und durch Umkristallisation aus Essigsäureethylester/Methanol unter Verwendung von Aktivkohle gereinigt.
Ausbeute: 19,8 g (46 % der Theorie)
Fp.: 223-224 °C (Essigsäureethylester/Methanol).

Beispiel 11

5,10-Dihydro-5-[[[2-(1-methyl-2-pyrrolidinyl)ethyl]methylamino]acetyl]-11H-dibenzo[b,e][1,4]diazepin-11-on-hydrochlorid

Hergestellt analog Beispiel 10 aus 5-(Chloracetyl)-5,10-dihydro-11H-dibenzo[b,e][1,4]diazepin-11-on und 1-Methyl-2-[2-(methylamino)ethyl]pyrrolidin.
Ausbeute: 42 % der Theorie.
Fp.: 208-210 °C (Essigsäureethylester/Methanol).

Beispiel 12

5,11-Dihydro-11-[[[2-(1-methyl-2-pyrrolidinyl)ethyl]ethylamino]acetyl]-6H-pyrido[2,3-b][1,4]benzodiazepin-6-on

Zu einer Lösung von 5,7 g (0,02 mol) 11-(Chloracetyl)-5,11-dihydro-6H-pyrido[2,3-b][1,4]benzodiazepin-6-on und 2,8 ml Triethylamin in 50 ml Dimethylformamid wurden 3,1 g (0,022 mol) 1-Methyl-2-[2-(ethylamino)ethyl]pyrrolidin getropft und die Mischung noch 0,5 Stunden bei Raumtemperatur gerührt. Nach dem Abdestillieren des Lösungsmittels wurde an Kieselgel chromatographiert (Mobile Phase: Methylenchlorid/Methanol 9/1 v/v). Die eingeengten Eluate wurden zwischen Kaliumcarbonat-Lösung und Essigsäureethylester verteilt. Nach Abdestillation des Lösungsmittels wurden die entstandenen Kristalle aus Diisopropylether/Essigsäureethylester umkristallisiert.
Ausbeute: 0,98 g (12 % der Theorie)
Fp.: 159-160 °C (Diisopropylether/Essigsäureethylester).

Beispiel 13

9-Chlor-5,11-dihydro-11-[[[2-(1-methyl-2-pyrrolidinyl)ethyl]ethylamino]acetyl]-6H-pyrido[2,3-b][1,4]-benzodiazepin-6-on

Eine Lösung von 3,2 g (0,01 mol) 9-Chlor-11-(chloracetyl)-5,11-dihydro-6H-pyrido[2,3-b][1,4]-benzodiazepin-6-on, 1,6 g (0,01 mol) 1-Methyl-2-[2-(ethylamino)ethyl]pyrrolidin und 3 g Kaliumcarbonat in 100 ml Acetonitril wurde 2 Stunden bei 60 °C gerührt, das Lösungsmittel im Vakuum abdestilliert, der Rückstand mit Wasser verrührt und mit Methylenchlorid extrahiert. Die nach säulenchromatographischer Reinigung an Kieselgel (Mobile Phase: Essigsäureethylester/Methanol/konz. Ammoniak 70/30/3 v/v/v) und Eindampfen der Eluate entstandenen Kristalle wurden aus Diisopropylether/Methanol umkristallisiert.
Ausbeute: 0,15 g (3,5 % der Theorie)
Fp.: 164-165 °C (Diisopropylether/Methanol).

Beispiel 14

5,10-Dihydro-5-[[[2-(1-methyl-2-pyrrolidinyl)ethyl]ethylamino]acetyl]-11H-dibenzo[b,e][1,4]diazepin-11-on

Hergestellt analog Beispiel 13 aus 5-(Chloracetyl)-5,10-dihydro-11H-dibenzo[b,e][1,4]diazepin-11-on und 1-Methyl-2-[2-(ethylamino)ethyl]pyrrolidin.
Ausbeute: 3,5 % der Theorie.
$R_F$ = 0,65 (Merck Fertigplatten, Kieselgel F 254; Fließmittel: Essigsäureethylester/Methanol/konz. Ammoniak 70/30/5 v/v/v).

Beispiel 15

6,11-Dihydro-11-[[[2-(1-methyl-2-pyrrolidinyl)ethyl]ethylamino]acetyl]-5H-pyrido[2,3-b][1,5]benzodiazepin-5-

on

Hergestellt analog Beispiel 13 aus 11-(Chloracetyl)-6,11-dihydro-5H-pyrido[2,3-b][1,5]benzodiazepin-5-on und 1-Methyl-2-[2-(ethylamino)ethyl]pyrrolidin.
Ausbeute: 13 % der Theorie.
Fp.: 139-140 °C (Diisopropylether/Essigsäureethylester/Aceton)

Im folgenden wird die Herstellung pharmazeutischer Anwendungsformen anhand einiger Beispiele beschrieben:

## Beispiel I

Tabletten mit 5 mg 5,11-Dihydro-11-[[[2-(1-methyl-2-pyrrolidinyl)ethyl]methylamino]acetyl]-6H-pyrido[2,3-b]-[1,4]benzodiazepin-6-on

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzukker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

## Beispiel II

Dragées mit 5 mg 5,11-Dihydro-11-[[[2-(1-methyl-2-pyrrolidinyl)ethyl]methylamino]acetyl]-6H-pyrido[2,3-b]-[1,4]benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

## Beispiel III

Ampullen mit 10 mg 5,11-Dihydro-11-[[[2-(1-methyl-2-pyrrolidinyl)ethyl]methylamino]acetyl]-6H-pyrido[2,3-b][1,4]benzodiazepin-6-on

| Zusammensetzung: | |
|---|---|
| 1 Ampulle enthält: | |
| Wirkstoff<br>Natriumchlorid<br>Dest. Wasser | 10,0 mg<br>8,0 mg<br>ad 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120 °C.

Beispiel IV

Suppositorien mit 20 mg 5,11-Dihydro-11-[[[2-(1-methyl-2-pyrrolidinyl)ethyl]methylamino]acetyl]-6H-pyrido-[2,3-b][1,4]benzodiazepin-6-on

| Zusammensetzung: | |
|---|---|
| 1 Zäpfchen enthält: | |
| Wirkstoff<br>Zäpfchenmasse (z.B. Witepsol W 45(R)) | 20,0 mg<br>1 680,0 mg<br>1 700,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.
Zäpfchengewicht 1,7 g

Beispiel V

Tropfen mit 5,11-Dihydro-11-[[[2-(1-methyl-2-pyrrolidinyl)ethyl]methylamino]acetyl]-6H-pyrido[2,3-b][1,4]-benzodiazepin-6-on

| Zusammensetzung: | |
|---|---|
| 100 ml Tropflösung enthalten: | |
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser | ad 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Ansprüche**

1.) Kondensierte Diazepinone der allgemeinen Formel I,

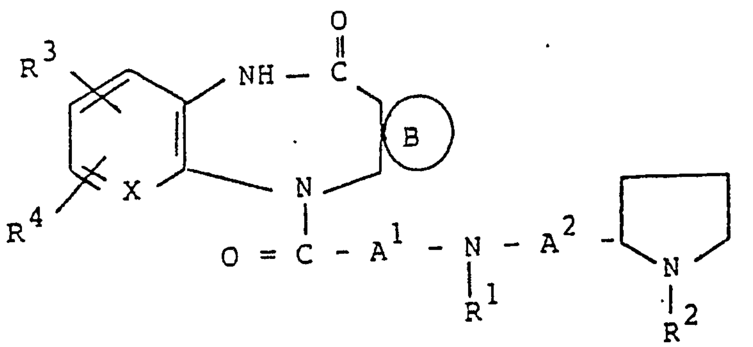

,(I)

in der 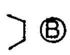 einen der zweiwertigen Reste

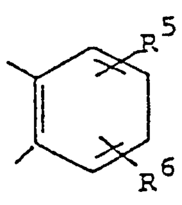

,

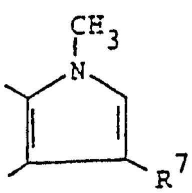

,

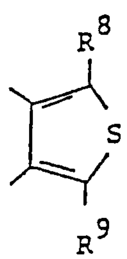

,

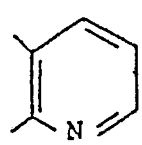

oder

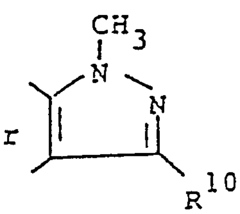

(S) (T) (U) (V) (W)

darstellt und
X, $A^1$, $A^2$ und $R^1$ bis $R^{10}$ die folgenden Bedeutungen besitzen:
X stellt eine =CH-Gruppe oder ein Stickstoffatom dar;
$A^1$ und $A^2$ sind geradkettige, gesättigte Alkylenreste mit 1 bis 4 Kohlenstoffatomen;
$R^1$ und $R^2$ sind Wasserstoffatome oder verzweigte oder unverzweigte Alkylreste mit 1 bis 4 Kohlenstoffatomen oder Cycloalkylreste mit 4 bis 7 Kohlenstoffatomen, die gegebenenfalls noch durch eine Hydroxygrup-

pe substituiert sein können;
$R^3$ ist eine Alkylgruppe mit 1 bis 4 C-Atomen, ein Chloratom oder ein Wasserstoffatom;
$R^4$ ein Wasserstoffatom oder eine Methylgruppe;
$R^5$ und $R^6$ bedeuten jeweils ein Fluor-, Chlor- oder Bromatom, eine Alkylgruppe mit 1 bis 4 C-Atomen oder, für den Fall, daß X eine =CH-Gruppe darstellt, auch ein Wasserstoffatom,
sofern X ein Stickstoffatom, $A^2$ eine geradkettige, gesättigte Alkylengruppe mit 2 bis 4 C-Atomen und $R^1$ ein Wasserstoffatom bedeuten, muß mindestens einer der Reste $R^5$ oder $R^6$ eine der obengenannten Bedeutungen mit Ausnahme der eines Wasser- stoffatoms innehaben, besitzt aber $R^1$ eine der obengenannten Bedeutungen mit Ausnahme der eines Wasserstoffatoms und ist $A^2$ eine geradkettige, gesättigte Alkylengruppe mit 2 bis 4 C-Atomen, so können die Reste $R^5$ und $R^6$ alle oben genannten Bedeutungen innehaben;
$R^7$ ein Wasserstoff- oder Chloratom oder eine Methylgruppe;
$R^8$ ein Wasserstoffatom oder einen Alkylrest mit 1 bis 4 Kohlenstoffatomen;
$R^9$ ein Wasserstoff- oder Halogenatom oder eine Alkylgruppe mit 1 bis 4 Kohlenstoffatomen und
$R^{10}$ ein Wasserstoffatom oder eine Methylgruppe, wobei in dem Fall, daß ]Ⓑ den zweiwertigen Rest (T) darstellt und $R^7$ ein Wasserstoffatom ist, $R^3$ kein Chloratom sein kann und in dem Fall, daß ]Ⓑ den zweiwertigen Rest (V) bedeutet, X kein Stickstoffatom ist,
gegebenenfalls ihre Isomeren bzw. Enantiomeren und ihre physiologisch verträglichen Säureadditionssalze mit anorganischen oder organischen Säuren.

2.) Als kondensierte Diazepinone die Verbindungen
5,11-Dihydro-11-[[[2-(1-methyl-2-pyrrolidinyl)ethyl]methylamino]acetyl]-6H-pyrido[2,3-b][1,4]benzodiazepin-6-on,
9-Chlor-5,11-dihydro-11-[[[2-(1-methyl-2-pyrrolidinyl)ethyl]ethylamino]acetyl]-6H-pyrido[2,3-b][1,4]-benzodiazepin-6-on und
5,10-Dihydro-5-[[[2-(1-methyl-2-pyrrolidinyl)ethyl]-methylamino]acetyl]-11H-dibenzo[b,e][1,4]diazepin-11-on
und deren physiologisch verträgliche Salze mit anorganischen oder organischen Säuren.

3.) Arzneimittel enthaltend eine oder mehrere Verbindungen gemäß Anspruch 1 oder 2 neben üblichen Träger- und/oder Hilfsstoffen.

4.) Verwendung der kondensierten Diazepinone gemäß Anspruch 1 und 2 und ihrer physiologisch verträglichen Salze zur Behandlung von Bradycardien und Bradyarrhythmien und von Spasmen an Colon, Blase und Bronchien.

5.) Verwendung der kondensierten Diazepinone gemäß Anspruch 1 und 2 und ihrer physiologisch verträglichen Salze zur Herstellung von Arzneimitteln zur Behandlung von Bradycardien und Bradyarrhythmien und von Spasmen an Colon, Blase und Bronchien.

6.) Verfahren zur Herstellung von kondensierten Diazepinonen der allgemeinen Formel I,

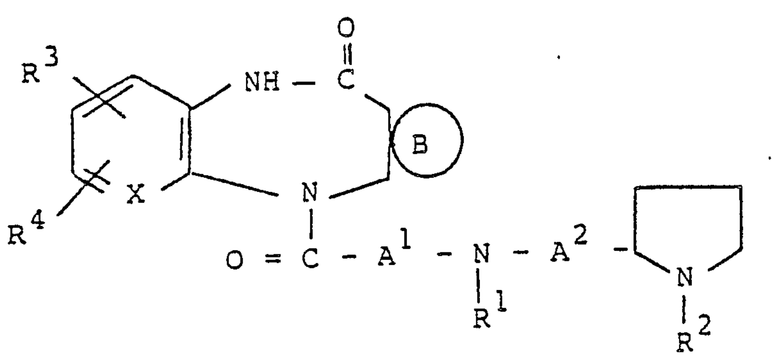

, (I)

in der ]Ⓑ einen der zweiwertigen Reste

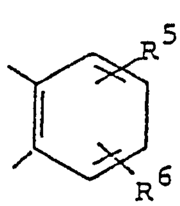 , 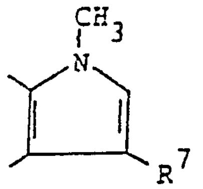 , 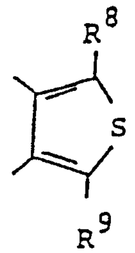 , 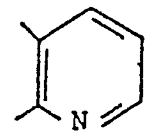 oder 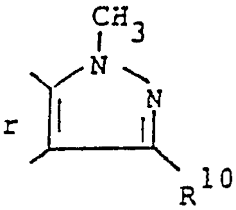

(S) (T) (U) (V) (W)

darstellt und
X, $A^1$, $A^2$ und $R^1$ bis $R^{10}$ die folgenden Bedeutungen besitzen:
X stellt eine =CH-Gruppe oder ein Stickstoffatom,
$A^1$ und $A^2$ geradkettige, gesättigte Alkylenreste mit 1 bis 4 Kohlenstoffatomen,
$R^1$ und $R^2$ Wasserstoffatome oder verzweigte oder unverzweigte Alkylreste mit 1 bis 4 Kohlenstoffatomen oder Cycloalkylreste mit 4 bis 7 Kohlenstoffatomen, die gegebenenfalls noch durch eine Hydroxygruppe substituiert sein können,
$R^3$ eine Alkylgruppe mit 1 bis 4 C-Atomen, ein Chloratom oder ein Wasserstoffatom,
$R^4$ ein Wasserstoffatom oder eine Methylgruppe,
$R^5$ und $R^6$ bedeuten jeweils ein Fluor-, Chlor- oder Bromatom, eine Alkylgruppe mit 1 bis 4 C-Atomen oder, für den Fall, daß X eine =CH-Gruppe darstellt, auch ein Wasserstoffatom,
sofern X ein Stickstoffatom, $A^2$ eine geradkettige, gesättigte Alkylengruppe mit 2 bis 4 C-Atomen und $R^1$ ein Wasserstoffatom bedeuten, muß mindestens einer der Reste $R^5$ oder $R^6$ eine der obengenannten Bedeutungen mit Ausnahme der eines Wasser- stoffatoms innehaben, besitzt aber $R^1$ eine der obengenannten Bedeutungen mit Ausnahme der eines Wasserstoffatoms und ist $A^2$ eine geradkettige, gesättigte Alkylengruppe mit 2 bis 4 C-Atomen, so können die Reste $R^5$ und $R^6$ alle oben genannten Bedeutungen innehaben;
$R^7$ ein Wasserstoff- oder Chloratom oder eine Methylgruppe,
$R^8$ ein Wasserstoffatom oder einen Alkylrest mit 1 bis 4 Kohlenstoffatomen,
$R^9$ ein Wasserstoff- oder Halogenatom oder eine Alkylgruppe mit 1 bis 4 Kohlenstoffatomen und
$R^{10}$ ein Wasserstoffatom oder eine Methylgruppe dar, wobei in dem Fall, daß ] (B) den zweiwertigen Rest (T) darstellt und $R^7$ ein Wasserstoffatom ist, $R^3$ kein Chloratom sein kann und in dem Fall, daß ] (B) den zweiwertigen Rest (V) bedeutet, X kein Stickstoffatom ist,
und von ihren möglichen Isomeren bzw. Enantiomeren und von ihren Säureadditionssalzen mit anorganischen oder organischen Säuren, dadurch gekennzeichnet, daß
a.) zur Herstellung von basisch substituierten kondensierten Diazepinonen der allgemeinen Formel Ia,

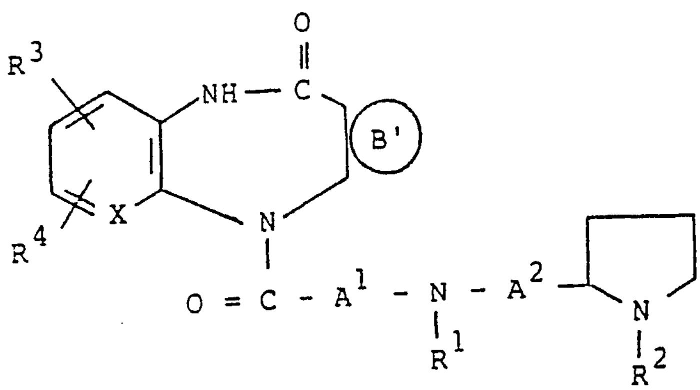

,(Ia)

in der
X, $A^1$, $A^2$ und $R^1$ bis $R^4$ wie oben definiert sind und ] (B) einen der zweiwertigen Reste (S), (U), (V), (W) oder (T′)

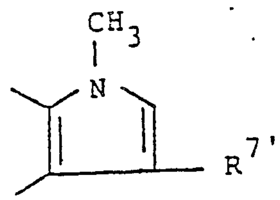

(T')

darstellt, worin $R^{7'}$ ein Chloratom oder eine Methylgruppe ist, Halogenacylverbindungen der allgemeinen Formel II,

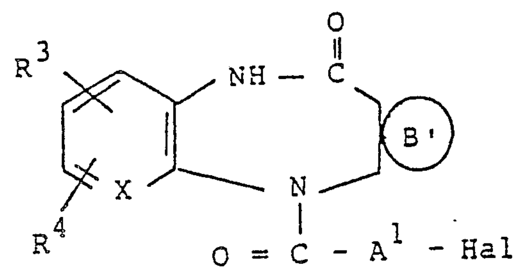

,(II)

in der
X, $A^1$, $R^3$, $R^4$ und 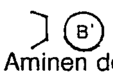 die vorstehend genannten Bedeutungen haben und Hal ein Chlor-, Brom- oder Iodatom ist, mit Aminen der allgemeinen Formel III,

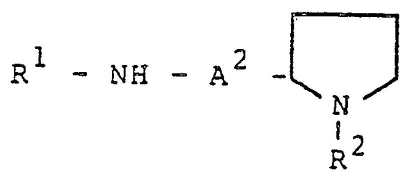

,(III)

worin $R^1$, $R^2$ und $A^2$ die genannten Bedeutungen haben, in einem Lösungsmittel bei Temperaturen zwischen -10 °C und der Siedetemperatur des Reaktionsgemisches, vorzugsweise in Gegenwart einer Hilfsbase oder eines Überschusses des Amins der allgemeinen Formel III, umgesetzt werden, oder
b.) zur Herstellung von basisch substituierten Diazepinonen der allgemeinen Formel Ia,
Diazepinone der allgemeinen Formel IV,

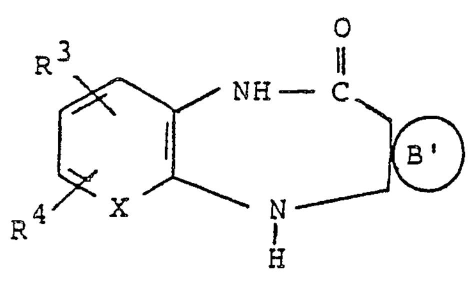

,(IV)

worin X, $R^3$, $R^4$ und 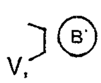 die oben angegebenen Bedeutungen haben, mit Carbonsäurederivaten der allgemeinen Formel V,

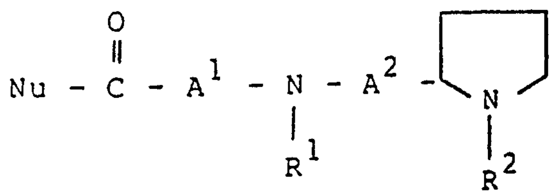

,(V)

worin $A^1$, $A^2$, $R^1$ und $R^2$ wie oben definiert sind und Nu eine nucleofuge Gruppe bzw. Abgangsgruppe darstellt, in einem inerten Lösungsmittel bei Temperaturen zwischen -25°C und 130°C umgesetzt werden, oder

c) zur Herstellung von unter die allgemeine Formel I fallenden Pyrrolo-kondensierten Diazepinonen der allgemeinen Formel Ib,

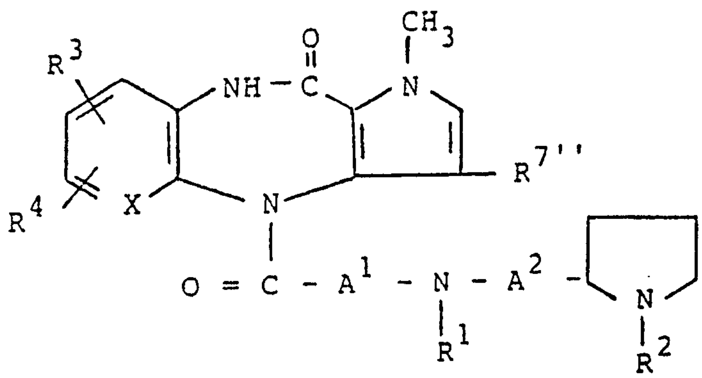

,(Ib)

worin

X, $A^1$, $A^2$ und $R^1$ bis $R^4$ die oben erwähnten Bedeutungen haben, und $R^{7''}$ ein Wasserstoffatom darstellt, Verbindungen der allgemeinen Formel Ib, in der $R^7$ für den Rest $R^7$ steht, wobei $R^7$ ein Chloratom bedeutet, in Anwesenheit eines Lösungsmittels bei Temperaturen zwischen 0°C und 130°C und in Gegenwart von Katalysatoren der Metalle der VIII. Nebengruppe des Periodensystems bei Wasserstoffdrukken von 1 bis 300 bar, oder, sofern der Wasserstoff durch Ameisensäure oder Triethylammoniumformiat in Gegenwart von Triethylamin ersetzt wird, drucklos einer Hydrogenolyse unterworfen werden,

die erhaltenen Verbindungen der allgemeinen Formel I gegebenenfalls anschließend in ihre Isomeren bzw. Enantiomeren aufgetrennt und/oder die erhaltenen Salze in die freien Ba sen und/oder die erhaltenen Basen in ihre physiologisch verträglichen Salze mit anorganischen oder organischen Säuren übergeführt werden.

7.) Verfahren gemäß Anspruch 6b, dadurch gekennzeichnet, daß als reaktive Carbonsäurederivate der allgemeinen Formel V deren Säurehalogenide, -ester, -anhydride oder gemischte Anhydride, insbesondere deren gemischte Anhydride mit starken Mineralsäuren verwendet werden, die Reaktion in Gegenwart von säurebindenden Mitteln durchgeführt wird und als inerte Lösungsmittel chlorierte aliphatische Kohlenwasserstoffe, offenkettige oder cyclische Ether, aromatische Kohlenwasserstoffe oder polare aprotische Lösungsmittel oder Gemische dieser Lösungsmittel eingesetzt werden.

8.) Verfahren gemäß Anspruch 6c, dadurch gekennzeichnet, daß die Hydrogenolyse

a.) mit Palladium auf Tierkohle oder auf Bariumsulfat, mit Raney-Nickel oder Raney-Cobalt als Katalysatoren und in Gegenwart von Alkoholen, Ethern, Carbonsäuren oder tertiären Aminen als Lösungsmitteln oder

b.) mit Ameisensäure, gegebenenfalls in Gegenwart von Dimethylformamid als Lösungsmittel und von Palladium auf Kohle als Katalysator bei Temperaturen zwischen 70 und 110°C, oder

c.) mit Triethylammoniumformiat in Gegenwart überschüssigen Triethylamins und von Palladium auf Tierkohle oder von Palladiumacetat und Triarylphosphinen bei Temperaturen zwischen 40 und 110°C durchgeführt wird.

Patentansprüche für folgende Vertragsstaaten:ES, GR

1.) Verfahren zur Herstellung von kondensierten Diazepinonen der allgemeinen Formel I,

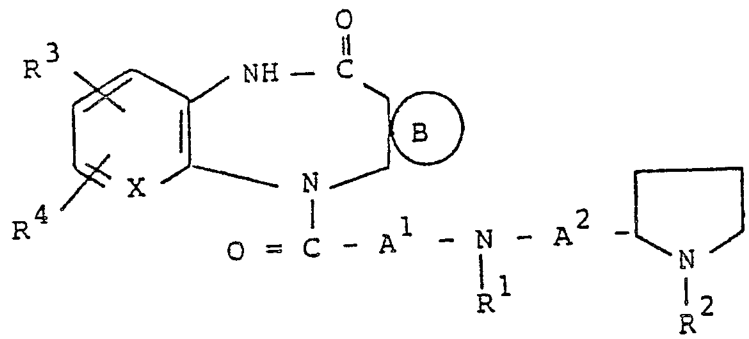

,(I)

in der 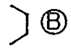 einen der zweiwertigen Reste

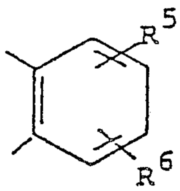 , 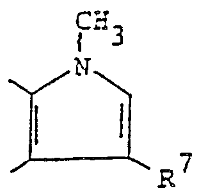 , 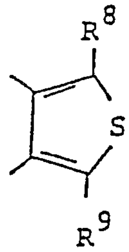 , 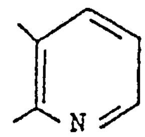 oder 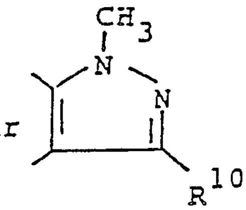

(S) (T) (U) (V) (W)

darstellt und

X, A1, $A^2$ und $R^1$ bis $R^{10}$ die folgenden Bedeutungen besitzen:

X stellt eine =CH-Gruppe oder ein Stickstoffatom,

$A^1$ und $A^2$ geradkettige, gesättigte Alkylenreste mit 1 bis 4 Kohlenstoffatomen,

$R^1$ und $R^2$ Wasserstoffatome oder verzweigte oder unverzweigte Alkylreste mit 1 bis 4 Kohlenstoffatomen oder Cycloalkylreste mit 4 bis 7 Kohlenstoffatomen, die gegebenenfalls noch durch eine Hydroxygruppe substituiert sein können,

$R^3$ eine Alkylgruppe mit 1 bis 4 C-Atomen, ein Chloratom oder ein Wasserstoffatom,

$R^4$ ein Wasserstoffatom oder eine Methylgruppe,

$R^5$ und $R^6$ bedeuten jeweils ein Fluor-, Chlor- oder Bromatom, eine Alkylgruppe mit 1 bis 4 C-Atomen oder, für den Fall, daß X eine =CH-Gruppe darstellt, auch ein Wasserstoffatom,

sofern X ein Stickstoffatom, $A^2$ eine geradkettige, gesättigte Alkylengruppe mit 2 bis 4 C-Atomen und $R^1$ ein Wasserstoffatom bedeuten, muß mindestens einer der Reste $R^5$ oder $R^6$ eine der obengenannten Bedeutungen mit Ausnahme der eines Wasser- stoffatoms innehaben, besitzt aber $R^1$ eine der obengenannten Bedeutungen mit Ausnahme der eines Wasserstoffatoms und ist $A^2$ eine geradkettige, gesättigte Alkylengruppe mit 2 bis 4 C-Atomen, so können die Reste $R^5$ und $R^6$ alle oben genannten Bedeutungen innehaben;

$R^7$ ein Wasserstoff- oder Chloratom oder eine Methylgruppe,

$R^8$ ein Wasserstoffatom oder einen Alkylrest mit 1 bis 4 Kohlenstoffatomen,

$R^9$ ein Wasserstoff- oder Halogenatom oder eine Alkylgruppe mit 1 bis 4 Kohlenstoffatomen und

$R^{10}$ ein Wasserstoffatom oder eine Methylgruppe dar, wobei in dem Fall, daß 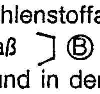 den zweiwertigen Rest (T) darstellt und $R^7$ ein Wasserstoffatom ist, $R^3$ kein Chloratom sein kann und in dem Fall, daß 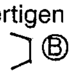 den zweiwertigen Rest (V) bedeutet, X kein Stickstoffatom ist,

und von ihren möglichen Isomeren bzw. Enantiomeren und von ihren Säureadditionssalzen mit anorganischen oder organischen Säuren, dadurch gekennzeichnet, daß

a.) zur Herstellung von basisch substituierten kondensierten Diazepinonen der allgemeinen Formel Ia,

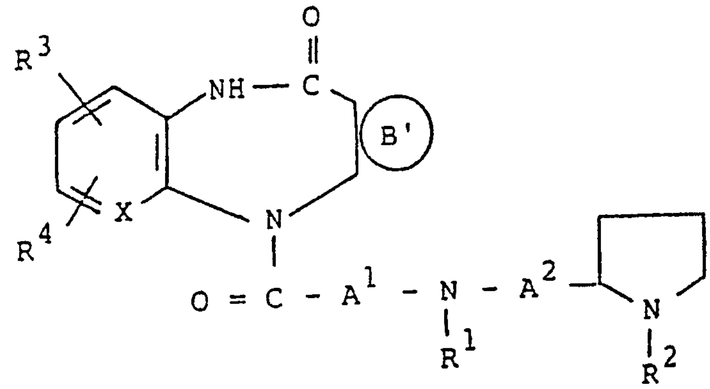

,(Ia)

in der
X, $A^1$, $A^2$ und $R^1$ bis $R^4$ wie oben definiert sind und 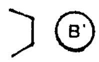 einen der zweiwertigen Reste (S), (U), (V), (W) oder (T′)

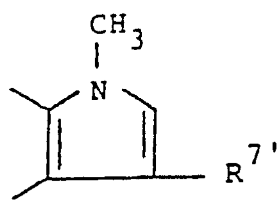

(T′)

darstellt, worin $R^{7'}$ ein Chloratom oder eine Methylgruppe ist, Halogenacylverbindungen der allgemeinen Formel II,

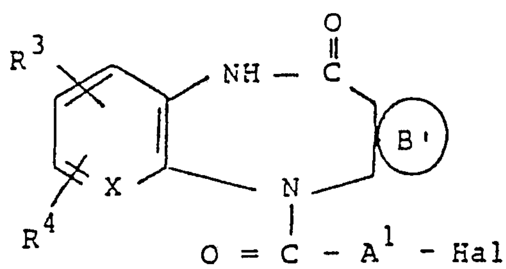

,(II)

in der
X, $A^1$, $R^3$, $R^4$ und 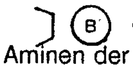 die vorstehend genannten Bedeutungen haben und Hal ein Chlor-, Brom- oder Iodatom ist, mit Aminen der allgemeinen Formel III,

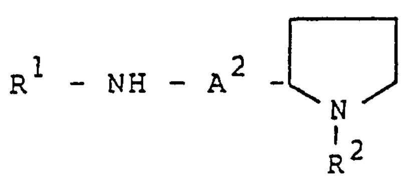

,(III)

worin $R^1$, $R^2$ und $A^2$ die genannten Bedeutungen haben, in einem Lösungsmittel bei Temperaturen zwischen -10 °C und der Siedetemperatur des Reaktionsgemisches, vorzugsweise in Gegenwart einer Hilfsbase oder eines Überschusses des Amins der allgemeinen Formel III, umgesetzt werden, oder
b.) zur Herstellung von basisch substituierten Diazepinonen der allgemeinen Formel Ia,
Diazepinone der allgemeinen Formel IV,

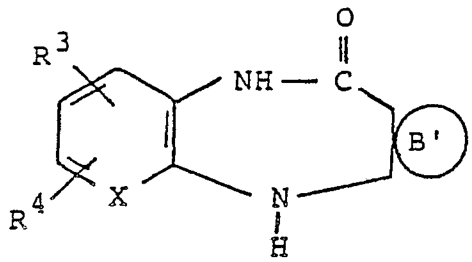

,(IV)

worin X, $R^3$, $R^4$ und 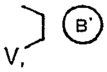 die oben angegebenen Bedeutungen haben, mit Carbonsäurederivaten der allgemeinen Formel V,

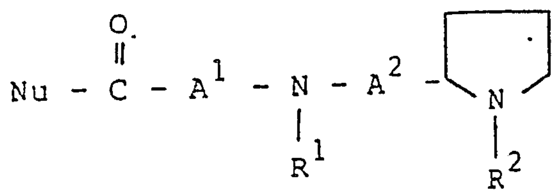

,(V)

worin $A^1$, $A^2$, $R^1$ und $R^2$ wie oben definiert sind und Nu eine nucleofuge Gruppe bzw. Abgangsgruppe darstellt, in einem inerten Lösungsmittel bei Temperaturen zwischen -25 °C und 130 °C umgesetzt werden, oder

c) zur Herstellung von unter die allgemeine Formel I fallenden Pyrrolo-kondensierten Diazepinonen der allgemeinen Formel Ib,

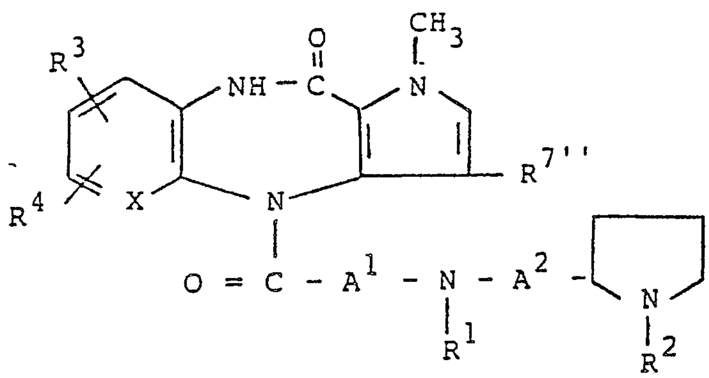

,(Ib)

worin

X, $A^1$, $A^2$ und $R^1$ bis $R^4$ die oben erwähnten Bedeutungen haben, und $R^{7''}$ ein Wasserstoffatom darstellt, Verbindungen der allgemeinen Formel Ib, in der $R^7$ für den Rest $R^7$ steht, wobei $R^7$ ein Chloratom bedeutet, in Anwesenheit eines Lösungsmittels bei Temperaturen zwischen 0 °C und 130 °C und in Gegenwart von Katalysatoren der Metalle der VIII. Nebengruppe des Periodensystems bei Wasserstoffdrukken von 1 bis 300 bar, oder, sofern der Wasserstoff durch Ameisensäure oder Triethylammoniumformiat in Gegenwart von Triethylamin ersetzt wird, drucklos einer Hydrogenolyse unterworfen werden,

die erhaltenen Verbindungen der allgemeinen Formel I gegebenenfalls anschließend in ihre Isomeren bzw. Enantiomeren aufgetrennt und/oder die erhaltenen Salze in die freien Basen und/oder die erhaltenen Basen in ihre physiologisch verträglichen Salze mit anorganischen oder organischen Säuren übergeführt werden.

2.) Verfahren gemäß Anspruch 1b, dadurch gekennzeichnet, daß als reaktive Carbonsäurederivate der allgemeinen Formel V deren Säurehalogenide, -ester, -anhydride oder gemischte Anhydride, insbesondere deren gemischte Anhydride mit starken Mineralsäuren verwendet werden, die Reaktion in Gegenwart von säurebindenden Mitteln durchgeführt wird und als inerte Lösungsmittel chlorierte aliphatische Kohlenwasserstoffe, offenkettige oder cyclische Ether, aromatische Kohlenwasserstoffe oder polare aprotische Lösungsmittel oder Gemische dieser Lösungsmittel eingesetzt werden.

3.) Verfahren gemäß Anspruch 1c, dadurch gekennzeichnet, daß die Hydrogenolyse

a.) mit Palladium auf Tierkohle oder auf Bariumsulfat, mit Raney-Nickel oder Raney-Cobalt als Katalysatoren und in Gegenwart von Alkoholen, Ethern, Carbonsäuren oder tertiären Aminen als Lösungsmitteln oder

b.) mit Ameisensäure, gegebenenfalls in Gegenwart von Dimethylformamid als Lösungsmittel und von Palladium auf Kohle als Katalysator bei Temperaturen zwischen 70 und 110 °C, oder

c.) mit Triethylammoniumformiat in Gegenwart überschüssigen Triethylamins und von Palladium auf Tierkohle oder von Palladiumacetat und Triarylphosphinen bei Temperaturen zwischen 40 und 110 °C durchgeführt wird.

Europäisches Patentamt

# EUROPÄISCHER TEILRECHERCHENBERICHT,
der nach Regel 45 des Europäischen Patentübereinkommens für das weitere Verfahren als europäischer Recherchenbericht gilt

Nummer der Anmeldung: EP 89110264.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A2/A3 - 0 057 428 (BYK GULDEN LOMBERG) * Zusammenfassung * -- | 1,3,5, 6 | C 07 D 471/04<br>C 07 D 471/14<br>C 07 D 243/38<br>C 07 D 487/04<br>C 07 D 495/04<br>C 07 D 495/14<br>C 07 D 403/12<br>A 61 K 31/55 |
| D,A | EP - A1 - 0 039 519 (BYK GULDEN LOMBERG) * Zusammenfassung * -- | 1,3,5, 6 | |
| A | US - A - 4 210 648 (SCHMIDT) * Zusammenfassung * -- | 1,3,5, 6 | |
| A | EP - A2 - 0 254 955 (DR. KARL THOMAE) * Zusammenfassung * -- | 1,3,5, 6 | |
| P,A | EP - A1 - 0 273 239 (DR. KARL THOMAE) * Zusammenfassung * -- | 1,3,5, 6 | |
| A | EP - A2/A3 - 0 213 293 (DR. KARL THOMAE) * Zusammenfassung * ---- | 1,3,5, 6 | |

RECHERCHIERTE SACHGEBIETE (Int. Cl.4)

C 07 D 471/00
C 07 D 243/00
C 07 D 487/00
C 07 D 495/00
C 07 D 403/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5-8

Unvollständig recherchierte Patentansprüche: –

Nicht recherchierte Patentansprüche: 4

Grund für die Beschränkung der Recherche:

(Artikel 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 21-08-1989 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82